# EUROPEAN PATENT SPECIFICATION

(11) **EP 4 248 848 B1**
(45) Date of publication and mention of the grant of the patent: **05.11.2025**
(21) Application number: 23186910.8
(22) Date of filing: 29.04.2021
(51) Int. Cl.: A61B 5/00

(54) **DEVICES AND SYSTEMS FOR DIAGNOSING TREATING AND MONITORING CHRONIC PELVIC PAIN**
VORRICHTUNGEN UND SYSTEME ZUR DIAGNOSE DER BEHANDLUNG UND ÜBERWACHUNG CHRONISCHER BECKENSCHMERZEN
DISPOSITIFS ET SYSTÈMES DE DIAGNOSTIC ET DE SURVEILLANCE DE LA DOULEUR PELVIENNE CHRONIQUE

(30) Priority: 30.04.2020 US 202063017921 P; 23.10.2020 US 202063104614 P; 11.12.2020 US 202063124664 P
(43) Date of publication of application: 27.09.2023
(62) Divisional of application: 21795350.4
(73) Proprietor: University of Houston System, Houston, Texas 77204 (US)
(72) Inventor: Zhang, Yingchun, Katy, 77450 (US); Dias, Nicholas, Houston, 77062 (US); Liu, Yang, Sugar Land, 77479 (US); Lai, Henry, Seattle, 98195 (US); Spitznagle, Theresa M., Manchester, 63021 (US); Smith, Christopher P., Missouri City, 77459 (US); Houston, Michael, Houston, 77067 (US)
(74) Representative: Schrell, Andreas

(56) References cited:
- WO-A1-2019/200222
- US-A1- 2003 100 930
- US-A1- 2019 247 650

## Description

### FIELD

The present disclosure relates to chronic pelvic pain, more specifically to devices, systems, and methods for diagnosing, treating, and monitoring for chronic pelvic pain.

### BACKGROUND

Chronic pelvic pain (CPP) is defined as persistent pain in the lower abdomen or the pelvis without an obvious on-going disease process. It is estimated to affect up to 20% of women in the US. CPP in women can be difficult to sort out due to overlapping clinical presentations and ill-defined symptoms and physical examination findings.

Current treatment regimens, such as physical therapy (PT) regimens, are largely unsuccessful in a significant portion of individuals suffering from CPP. Carrying out PT regimens is a time-consuming process for the healthcare providers and patients, and thus there is an opportunity to significantly improve PT regimens on an individual basis to achieve higher treatment success rates Documents US 2019/247650 and WO 2019/200222 A1 disclose therapeutic aspects for treating muscles disorders e.g. CPP.

### SUMMARY

The invention is as defined in the appended claims. Provided in accordance with aspects of the present disclosure is a system for determining a treatment regimen for chronic pelvic pain (CPP). The system includes an electromyography (EMG) probe including electrodes. Each electrode detects pelvic floor muscle activity of a body of a patient. An EMG sensor set includes bipolar EMG sensors configured to be arranged about the body of the patient. Each of the bipolar EMG sensors detects muscle activity of muscles connected to the pelvic floor muscles of the body of the patient. An EMG amplifier is in communication with the EMG probe or the EMG sensor set. The EMG amplifier includes a plurality of input channels. Each input channel receives data of muscle activity in the pelvic floor muscles or the muscles connected to the pelvic floor muscles from the EMG probe or the EMG sensor set. A computer is in communication with the EMG amplifier. The computer includes a processor and a memory. The memory stores computer instructions configured to be executed by the processor. The computer instructions instruct the processor to perform muscle network analysis using the data of muscle activity in the pelvic floor muscles or the muscles connected to the pelvic floor muscles. A treatment regimen for CPP in the patient is recommended based on the muscle network analysis.

In an aspect of the present disclosure, a first wireless module is connected to the EMG probe. A second wireless module is connected to the EMG sensor set. A third wireless module is connected to the EMG amplifier. Each of the first, second, and third wireless modules is configured to transmit or receive the data of muscle activity in the pelvic floor muscles or the muscles connected to the pelvic floor muscles.

In an aspect of the present disclosure, the EMG probe includes sensor bands. Each of the senor bands includes spaced apart sensors arranged circumferentially around the EMG probe. A fin is positioned at a proximal end portion of the EMG probe opposite a distal end portion of the EMG probe. The fin is aligned with a sensor of the spaced apart sensors to determine a directional orientation of the sensor with respect to the body of the patient.

In an aspect of the present disclosure, the EMG probe includes at least 36 electrodes configured to detect pelvic floor muscle activity. The EMG sensor set includes at least 20 bipolar EMG sensors configured to detect muscle activity in hip muscles, leg muscles, back muscles, or abdominal muscles. The input channels of the EMG amplifier include at least 36 input channels in respective communication with the at least 36 electrodes configured to detect pelvic muscle activity. The input channels of the EMG amplifier include at least 20 input channels in respective communication with the at least 20 bipolar EMG sensors configured to detect muscle activity in hip muscles, leg muscles, back muscles, or abdominal muscles.

In an aspect of the present disclosure, the recommended treatment regimen for CPP is a physical therapy (PT) treatment. The PT treatment includes myofascial therapy or movement training.

In an aspect of the present disclosure, the performed muscle network analysis identifies hypertonicity in at least one muscle of the patient indicative of CPP.

In an aspect of the present disclosure, the computer instructions instruct the processor to perform an inter-muscle coherence analysis using the data of muscle activity in the pelvic floor muscles or the muscles connected to the pelvic floor muscles of the body of the patient.

In an aspect of the present disclosure, the data of muscle activity in the pelvic floor muscles or the muscles connected to the pelvic floor muscles is received in the EMG amplifier at substantially the same time that the muscle network analysis is performed.

In an aspect of the present disclosure, the muscle network analysis is performed in real-time while a PT treatment is performed on the patient. The computer instructions instruct the processor to perform a second muscle network analysis during the PT treatment and recommend a second treatment regimen for CPP in the patient based on the second muscle network analysis.

Provided in accordance with aspects of the present disclosure is a system for monitoring a treatment regimen for CPP including an EMG sensor set including a plurality of surface EMG sensors configured to capture data of muscle activity in hip muscles, leg muscles, back muscles, or abdominal muscles of a patient performing a physical therapy treatment regimen for CPP. The physical therapy treatment regimen for CPP is based on abnormal Pelvic Floor Muscle (PFM) to Hip/Trunk muscle connections. A computer is in communication with the EMG sensor set. The computer is configured to receive the captured data from the EMG sensor set. The computer includes a processor and a memory. The memory stores computer instructions configured to be executed by the processor. The computer instructions instruct the processor to perform a muscle activation pattern analysis and an inter-muscle interaction pattern analysis using the captured data from the EMG sensor set, and determine if a modification is needed to the physical therapy treatment regimen based at least on the muscle activation pattern analysis or the inter-muscle interaction pattern analysis.

In an aspect of the present disclosure, the computer is in communication with the EMG sensor set is a smartphone or tablet computer.

In an aspect of the present disclosure, a wireless module is in communication with a cloud-based server. The wireless module is configured to transmit the captured data from the EMG sensor set or the modification to the physical therapy treatment regimen for CPP to the cloud-based server for communication with a healthcare provider.

In an aspect of the present disclosure, the surface EMG sensors of the plurality of surface EMG sensors are wireless sensors configured to connect wirelessly with the computer.

In an aspect of the present disclosure, the physical therapy treatment regimen for CPP includes an at-home physical therapy regimen including a plurality of physical therapy sessions.

Provided in accordance with aspects of the present disclosure is a method of monitoring a treatment regimen for CPP including capturing data, by an EMG sensor set including a plurality of surface EMG sensors data of muscle activity in hip muscles, leg muscles, back muscles, or abdominal muscles of a patient performing a physical therapy treatment regimen for CPP. The physical therapy treatment regimen for CPP is based on abnormal PFM to Hip/Trunk muscle connections. The method includes receiving, by a computer, data from the EMG sensor set. The method includes performing, by the computer, a muscle activation pattern analysis and an inter-muscle interaction pattern analysis using the captured data from the EMG sensor set. The method includes determining, by the computer, if a modification is needed to the physical therapy treatment regimen based on the muscle activation pattern analysis and the inter-muscle interaction pattern analysis.

In an aspect of the present disclosure, the method includes determining, by the computer, a modification to an at-home physical therapy regimen including a plurality of physical therapy sessions.

In accordance with aspects of the present disclosure, a computer implemented method of pelvic muscle hypertonicity severity assessment and NJM mapping is presented. The method includes capturing a first high-density surface electromyography (HD-sEMG) signal, by an intra-vaginal probe, of pelvic muscle activity at rest; capturing a second HD-sEMG signal, by the intra-vaginal probe, of pelvic muscle activity during a voluntary contraction of the pelvic muscle; calculating a pelvic muscle hypertonicity index based on the first HD-sEMG signal and the second HD-sEMG signal; performing HD-sEMG decomposition of the first HD-sEMG signal and the second HD-sEMG signal into motor unit action potentials (MUAP), by a HD-sEMG decomposition algorithm; assessing hypertonicity severity based on the pelvic muscle hypertonicity index; mapping the NMJ locations of the pelvic floor muscles based on the HD-sEMG decomposition; determining at least one botulinum neurotoxin (BoNT) injection site based on the NMJ map; and determining BoNT dosage for the at least one injection site based on the corresponding pelvic muscle hypertonicity index.

In an aspect of the present disclosure, the method may further include providing a personalized BoNT injection into the pelvic muscles based on the determined at least one BoNT injection site and the determined dosage.

In an aspect of the present disclosure, the method may further include diagnosing the pelvic floor hypertonicity based on the HD-sEMG decomposition.

In an aspect of the present disclosure, the intra-vaginal probe is configured for wireless communication with an EMG amplifier.

In accordance with aspects of the present disclosure, a system for pelvic muscle hypertonicity severity assessment and NJM mapping is presented. The system includes an EMG amplifier, an intra-vaginal configured for vaginal high-density surface electromyography (HD-sEMG) signal acquisition, the probe including a surface electrode grid, a processor, and a memory. The memory, has instructions stored thereon, which when executed by the processor cause the system to capture a first HD-sEMG signal, by an intra-vaginal probe, of pelvic muscle activity at rest; capture a second HD-sEMG signal, by the intra-vaginal probe, of pelvic muscle activity during a voluntary contraction of the pelvic muscles; calculate a pelvic muscle hypertonicity index based on the first HD-sEMG signal and the second HD-sEMG signal; perform HD-sEMG decomposition of the first HD-sEMG signal and the second HD-sEMG signal into MUAPs, by a HD-sEMG decomposition algorithm, based on the pelvic muscle hypertonicity index; map the NMJ locations over the pelvic floor muscles based on the HD-sEMG decomposition; determine BoNT at least one injection site based on the NMJ maps of the pelvic muscles; and determine BoNT dosage for the at least one injection site based on the corresponding pelvic muscle hypertonicity index.

In an aspect of the present disclosure, the instructions when executed may further cause the system to provide a personalized BoNT injection, based on the determined at least one BoNT injection site and the determined dosage, into the pelvic muscles.

In an aspect of the present disclosure, the instructions when executed may further cause the system to diagnose the pelvic floor hypertonicity based on spatiotemporal muscle activity information captured by HD-sEMG.

In an aspect of the present disclosure, the intra-vaginal probe is configured for wireless communication with an EMG amplifier.

### BRIEF DESCRIPTION OF THE DRAWINGS

Various aspects and features of the present disclosure are described hereinbelow with reference to the drawings wherein:
FIGS. 1A, 1B, and 1C illustrate a system for determining an individualized treatment regimen for chronic pelvic pain (CPP) according to aspects of the present disclosure;
FIG. 2 illustrates positioning of an EMG probe of the system of FIGS. 1A, 1B, and 1C in a patient;
FIG. 3A illustrates anterior positioning of EMG sensors of the system of FIGS. 1A, 1B, and 1C about a patient;
FIG. 3B illustrates posterior positioning of EMG sensors of the system of FIGS. 1A, 1B, and 1C about the patient;
FIG. 4A, 4B, and 4C illustrate exemplary muscle activity and inter-muscle coherence detected by the system of FIGS. 1A, 1B, and 1C;
FIG. 5 is a block diagram of a method for determining an individualized treatment regimen for CPP according to aspects of the present disclosure;
FIG. 6 is a block diagram of an exemplary computer of the system of FIGS. 1A, 1B, and 1C according to aspects of the present disclosure;
FIGS 7A and 7B illustrate a system for determining and monitoring an individualized at-home treatment regimen for chronic pelvic pain (CPP) according to aspects of the present disclosure;
FIG. 8 illustrates positioning of a probe of the system of FIGS. 7A and 7B within a patient;
FIG. 9A illustrates anterior positioning of EMG sensors of the system of FIGS. 7A and 7B;
FIG. 9B illustrates posterior positioning of EMG sensors of the system of FIGS. 7A and 7B;
FIG. 10A, 10B, and 10C illustrate exemplary muscle activity and inter-muscle coherence detected by the system of FIGS. 7A and 7B;
FIG. 11 illustrates an exemplary software interface of the system of FIGS. 7A and 7B.
FIG. 12 is a block diagram of a method of monitoring a treatment for CPP according to aspects of the present disclosure;
FIG. 13 is a perspective view of a vaginal high-density surface electromyography (HD-sEMG) evaluation apparatus, in accordance with aspects of the present disclosure;
FIG. 14 is a perspective view of a vaginal probe for the vaginal HD-sEMG evaluation apparatus of FIG. 13, in accordance with aspects of the present disclosure;
FIG. 15 is diagram of an exemplary controller, in accordance with aspects of the present disclosure;
FIG. 16 is a side view of the vaginal probe of FIG. 14 introduced into a vaginal space, in accordance with aspects of the present disclosure;
FIG. 17A is an illustration of an intra-vaginal HD-sEMG probe, in accordance with the present disclosure;
FIG. 17B is an illustration of the unwrapped 64-channel electrode grid with RMS mapping overlaid, in accordance with the present disclosure;
FIG. 17C is a graph of a recorded differential EMC signal, in accordance with the present disclosure;
FIG. 17D is an image of a bipolar map of a 64-channel MUAP after decomposition and spike triggered averaging, in accordance with aspects of the present disclosure;
FIG. 18A is an illustration of a testing protocol, in accordance with aspects of the present disclosure;
FIG. 18B is a table of EMG findings, in accordance with aspects of the present disclosure;
FIG. 19 is a graph of a resting RMS ratio for each diagnosis, in accordance with aspects of the present disclosure;
FIGS. 20A-B are an illustration of a 64-channel resting RMS for all subjects, in accordance with aspects of the present disclosure;
FIG. 21 is a graph illustrating PFM alignment as shortened, normal or lengthened, in accordance with aspects of the present disclosure;
FIG. 22A is a graph illustrating ability to lower PFM, in accordance with aspects of the present disclosure;
FIG. 22B is a graph illustrating ability to relax PFM, in accordance with aspects of the present disclosure;
FIGS. 23A-C are graphs illustrating linear relationships between resting RMS ratio and IC symptom index, IC problem index, and 0-10 pain scores, in accordance with aspects of the present disclosure;
FIGS. 24A-B are images of high-density EMG in pelvic floor hypertonicity, in accordance with aspects of the present disclosure; and
FIGS. 25A-B are top views of the vaginal probe of FIG. 14, in accordance with aspects of the present disclosure.

### DETAILED DESCRIPTION

As used herein, the term "distal" refers to the portion that is being described which is further from an operator (whether a human surgeon or a surgical robot), while the term "proximal" refers to the portion that is being described which is closer to the operator. The term "about" and the like, as utilized herein, are meant to account for manufacturing, material, environmental, use, and/or measurement tolerances and variations. Further, to the extent consistent, any of the aspects described herein may be used in conjunction with none, any, or all of the other aspects described herein.

Descriptions of technical features or aspects of an exemplary configuration of the disclosure should typically be considered as available and applicable to other similar features or aspects in another exemplary configuration of the disclosure. Accordingly, technical features described herein according to one exemplary configuration of the disclosure may be applicable to other exemplary configurations of the disclosure, and thus duplicative descriptions may be omitted herein.

Exemplary configurations of the disclosure will be described more fully below (e.g., with reference to the accompanying drawings). Like reference numerals may refer to like elements throughout the specification and drawings.

Chronic pelvic pain (CPP), defined herein as persistent pain in the lower abdomen or the pelvis without an obvious on-going disease process, is estimated to affect up to 20% of women in the US. Pelvic floor hypertonicity (PFH), characterized by an increase in the tonic activity of a pelvic floor muscle, is a symptom related to myofascial pain that presents in up to 85% of patients with interstitial cystitis/bladder pain syndrome (IC/BPS), up to 90% of vulvodynia, as well as a substantial portion of irritable bowel syndrome (IBS) and endometriosis. The etiology of PFH is associated with direct muscle injuries such as obstetric trauma, instrumental delivery, or pelvic surgery, as well as overuse injuries that can occur due to IBS, obstructive defecation, or anxiety. Multiple studies have shown that interventions for PFM impairments, including injections and myofascial massage, relieve pain. However, the underlying therapeutic mechanism remains poorly understood. Consequently, it is clinically important to objectively and quantitatively assess pelvic floor dysfunction in IC/BPS to better understand the etiology of PFH and ensure complete symptom resolution. Unfortunately, little effort has been made to assess the contribution of PFM innervation to PFH, possibly because of a lack of competent tools for PFM neuromuscular assessment.

CPP may manifest in women with hypertonic pelvic floor muscles. PFH, characterized by an increase in the tonic activity of a pelvic floor muscle, is a symptom related to myofascial pain that presents in many CPP conditions, including up to 85% of patients with interstitial cystitis/bladder pain syndrome (IC/BPS), up to 90% of vulvodynia, as well as a substantial portion of irritable bowel syndrome (IBS) and endometriosis. The etiology of PFH is associated with direct muscle injuries such as obstetric trauma, instrumental delivery or pelvic surgery, as well as overuse injuries, that can occur due to IBS, obstructive defecation or anxiety. In either case there is a consequent release of neuromuscular transmitters and inflammatory mediators, sensitization of the peripheral or central neural system, and finally the noxious perception of normal sensory input (allodynia) and myofascial pain. Clinical management of PFH involves the retraining and rehabilitation of the dysfunctional muscles, often through behavioral and physical therapy, oral medications, neuromodulation and trigger point injections.

Myofascial physical therapy (MPT) has become standard treatment among female CPP patients with concomitant pelvic floor tenderness. Unfortunately, even among a very specific IC/BPS patient population, only a 59% of patients reported improvement after treatment. The high non-responder rate to MPT may be explained by the incomplete understanding of multifactorial etiology of pelvic floor pain in women. MPT treats specifically the pelvic muscles, and aims to mobilize soft tissue via manual massage, relax the muscle via dilation, or improve muscle coordination with muscle retraining of the pelvic muscles to resolve somatic abnormalities causing pelvic floor pain. However, MPT does not address posture and movement impairments of the trunk and hip, which are associated with the presence of pelvic pain. Complementary to myofascial therapy, movement physical therapy is a treatment philosophy that aims to correct postural dysfunction and correct aberrant movement patterns that may cause pelvic pain. Pelvic floor pain is intrinsically a multifactorial dysfunction that can be attributed to postural issues, myofascial trigger points, peripheral sensitization, and abnormal muscle tone. Unfortunately, no technology is currently available for quantitatively and subjectively assessing the relative importance of these etiologic factors associated with pelvic floor pain, which, otherwise, would allow for 1) phenotyping patients for appropriate physical therapy intervention, and 2) personalizing physical therapeutic protocol. Physical therapy is a very time-consuming and labor-intensive protocol. Therefore, this 'try and see' strategy results in higher healthcare costs and frustration for treatment providers and patients.

There is no technique currently available for objectively and quantitatively assessing muscle activation pattern for individual muscles and inter-muscle interaction pattern for muscle pairs involved in the PFM-Hip-Trunk muscle network in real time and wirelessly. The PFM-Hip-Trunk muscle network consists of over 20 muscle groups including the pelvic floor muscles, hip muscles, leg muscles, back muscles and abdominal muscles which can all possibly contribute to chronic pelvic pain. The current standard treatment, myofascial physical therapy (MPT) treats specifically the pelvic muscles, and aims to mobilize soft tissue via manual massage, relax the muscle via dilation, or improve muscle coordination with muscle retraining of the pelvic muscles to resolve somatic abnormalities causing pelvic floor pain. However, MPT does not address posture and movement impairments of the trunk and hip, which are associated with the presence of pelvic pain. Complementary to myofascial therapy, movement physical therapy is a treatment philosophy that aims to correct postural dysfunction and correct aberrant movement patterns that may cause pelvic pain. Pelvic floor pain is intrinsically a multifactorial dysfunction that can be attributed to postural issues, myofascial trigger points, peripheral sensitization, and abnormal muscle tone.

Aspects of the preset disclosure include a novel intra-vaginal high-density surface electromyography system and method to reliably and quantitatively assess pelvic floor muscle hypertonicity, and a novel muscle network analysis system and method to reveal the inter-muscle coherence pattern alterations representing the neural drive from the central nervous system. Aspects of the present disclosure provide solutions for optimizing chronic pelvic pain (CPP) management via phenotyping patients for appropriate interventions (myofascial therapy or movement training) and personalizing physical therapy intervention guided using dynamic intermuscular interaction pattern from real-time muscle network analysis based on multi-channel surface electromyography (EMG) signals of the PFM-Hip- Trunk muscle network.

The systems and methods described herein according to aspects of the disclosure provide solutions for optimizing CPP management via phenotyping patients for appropriate intervention (myofascial therapy or movement training) and personalizing physical therapy intervention guided using dynamic intermuscular interaction pattern from real time muscle network analysis based on multi-channel surface EMG signals of the pelvic floor, hip, leg, back and abdominal muscles.

The systems and methods described herein according to aspects of the disclosure provide for creating the PFM-Hip-Trunk muscle network using wireless multi-channel surface EMG recording including the intra-vaginal high-density surface EMG recording (e.g., via at least 36 electrodes) and 20 bipolar surface EMG recording (e.g., via at least 20 bipolar EMG electrodes) from the hip muscles, leg muscles, back muscles and abdominal muscles which are functionally connected to pelvic floor muscles (PFM).

The systems and methods described herein according to aspects of the disclosure provide for objectively and quantitatively assessing muscle activation pattern for individual muscles and inter-muscle interaction pattern for muscle pairs involved in the PFM-Hip-Trunk muscle network in real time, from multi-channel surface EMG recordings via the inter-muscle synergy, intermuscle coherence and muscle network analysis.

The systems and methods described herein according to aspects of the disclosure provide for phenotyping CPP patients for appropriate physical therapy intervention using the objective and quantitative assessment of muscle activation pattern of individual muscles and inter-muscle interaction pattern of muscle pairs involved in the PFM-Hip-Trunk muscle network.

The systems and methods described herein according to aspects of the disclosure provide for creating an adaptive and personalized intervention platform by adaptively modifying physical therapy protocol during the multi-session training, guided by dynamic alterations of muscle activation patterns and inter-muscle interaction patterns of the PFM-Hip-Trunk muscle network.

The systems and methods described herein according to aspects of the disclosure provide for a novel solution for phenotyping CPP patients for appropriate physical therapy protocol for the optimized treatment outcome in CPP management.

The systems and methods described herein according to aspects of the disclosure provide for a novel solution for phenotyping CPP patients with a wireless multi-channel surface EMG recording system to protect patients' privacy.

The systems and methods described herein according to aspects of the disclosure provide for a novel solution for adaptive and personalized physical therapy training for the optimized treatment outcome in CPP management.

The systems and methods described herein according to aspects of the disclosure provide for a novel solution for adaptive and personalized physical therapy training with a wireless multichannel surface EMG recording system to protect patients' privacy.

Muscle networks represent a series of interactions among muscles in the central nervous system's effort to reduce the redundancy of the musculoskeletal system in motor-control. How this occurs has been investigated in healthy subjects with a novel technique exploring the functional connectivity between muscles through intermuscular coherence (IMC). Surface and internal EMG data can be collected to assess muscle activity and concurrent activity between functionally connected muscles. Muscle activity can be compared between normal individuals and individuals with CPP (i.e., "muscle network analysis") to characterize the disease state and progression of CPP, and to inform improved treatments regimens, and monitor the progress of ongoing treatment regimens.

Muscle networks describe the functional connectivity between muscles quantified using their associated intermuscular coherence, which reflect the descending neural control property. The functional interactions indicated by muscle networks reflect the effort of the central nervous system in reducing the redundancy of the musculoskeletal system in motor control. Alterations in functional interactions between muscles (i.e., "inter-muscle coherence analysis") can also be used to characterize the disease state and progression of CPP, and to inform improved treatments regimens, and monitor the progress of ongoing treatment regimens.

As an example, IMC can be computed for all muscle pairs to form a coherence matrix which is further decomposed using non-negative matrix factorization. The variance accounting for thresholding can then performed to identify the number of muscle networks and the common spectral patterns of coherence underlying the muscle networks. Coherence patterns can be converted to unit vectors and network adjacency matrices can be re-scaled using the coherence pattern vector norm prior to a network threshold. Identified patterns of alterations with respect to normal IMC can be used to identify assess CPP, as described herein.

An exemplary IMC calculation may be performed, as follows. A 0.5-second sliding time window with 50% overlap in the inter-muscle coherence calculation. The 0.5 second sliding time window is reduced from a generally applied 2-second sliding time window. The smaller time window is applied because performing pelvic floor contraction is different from performing arm/leg muscle contraction. For example, it is impractical to ask a woman to contract her pelvic muscle for > 10 seconds, the majority women can only contract for ~ 5 seconds. For arm/leg muscle contraction, patients can generally contract > 30 seconds.

Rescaling Adjacency: the purpose for rescaling adjacency matrices is to make the adjacency matrices comparable. Without a rescaling step, it might only be possible to identify muscle contraction patterns within a specific adjacency matrix, and it might not be possible to compare across all adjacency matrices for a patient. Thus, the rescaling step in the algorithms described herein allows evaluation of unique features associated with CPP.

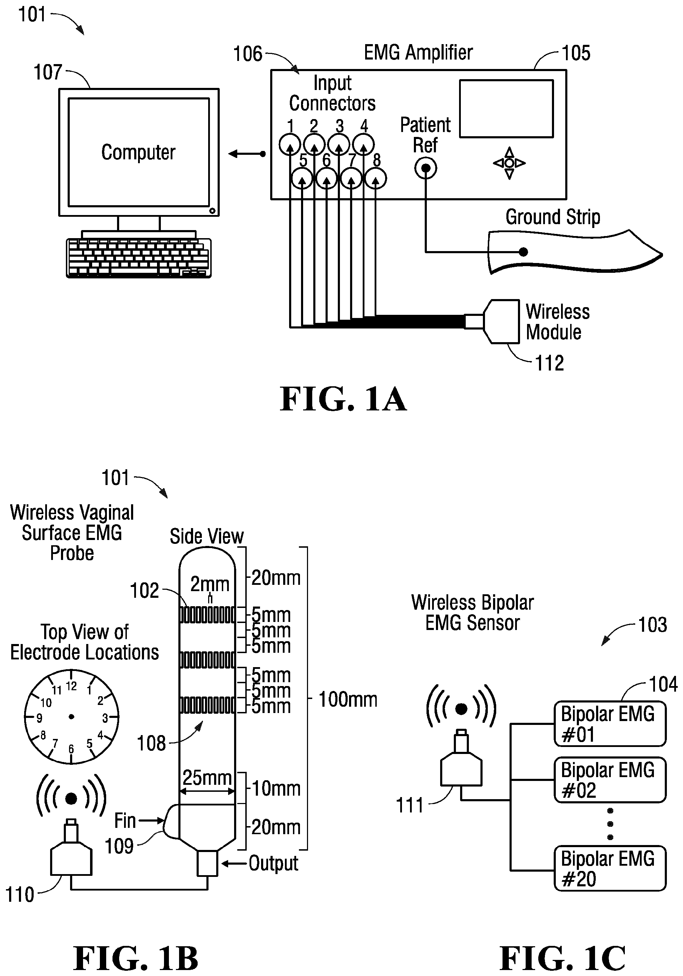

FIGS. 1A to 1C illustrate a multi-channel surface EMG apparatus for the PFMHip-Trunk muscle network evaluation. FIG. 1A shows the multi-channel wireless EMG recording amplifier; FIG. 1B shows the vaginal high-density surface EMG probe with a wireless module, and FIG. 3C shows the bipolar EMG sensors with a wireless module.

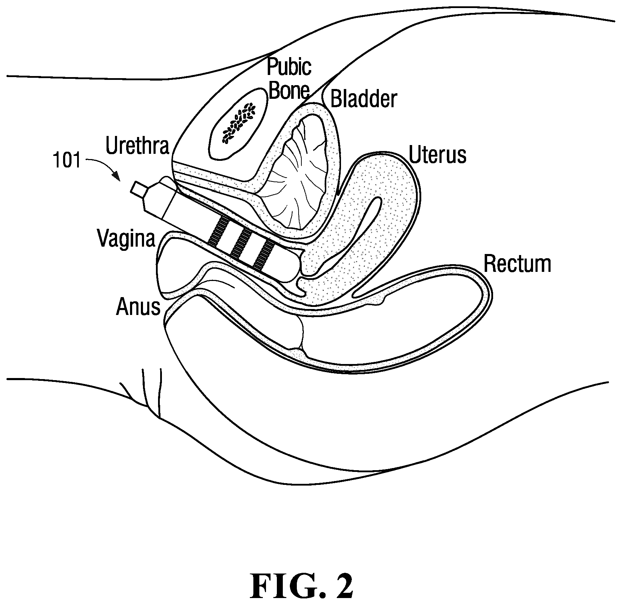

FIG. 2 illustrates the use of disclosed vaginal high-density surface EMG probe for EMG recordings from the pelvic floor muscles.

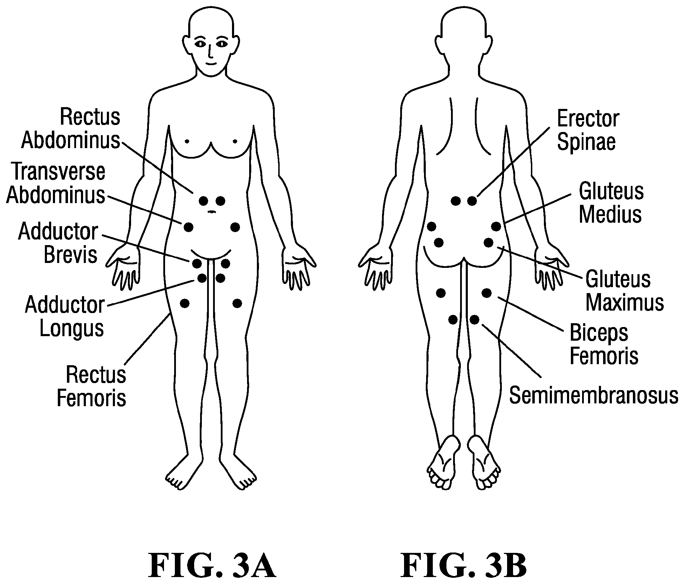

FIGS. 3A and 3B illustrate use of disclosed wireless bipolar surface EMG sensors for EMG recordings from the hip muscles, leg muscles, back muscles, and abdominal muscles. The systems and methods according to aspects of the disclosure provide for objectively and quantitatively assessing muscle activation pattern for individual muscles and inter-muscle interaction pattern for muscle pairs involved in the PFM-Hip-Trunk muscle network in real time and wirelessly.

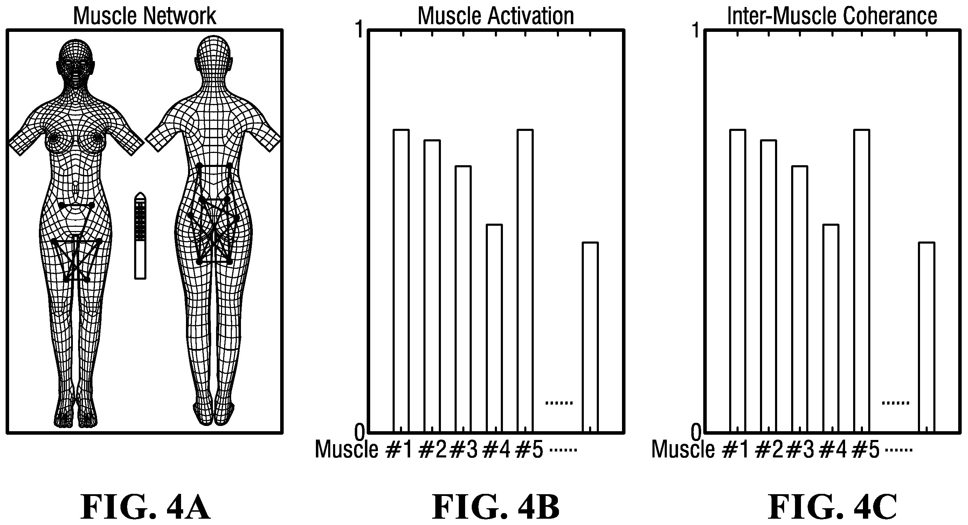

FIGS. 4A to 4C illustrate exemplary muscle activation patterns for individual muscles and intermuscle interaction pattern for muscle pairs involved in the PFM-Hip-Trunk muscle network, which change in real time and will be used to guide adaptive and personified physical training.

Referring to FIGS. 1A to 4C, a system 100 for determining a treatment for CPP includes an electromyography (EMG) probe 101 including electrodes 102. Each electrode 102 detects pelvic floor muscle activity of a body of a patient. An EMG sensor set 103 includes bipolar EMG sensors 104 configured to be arranged about the body of the patient. Each of the bipolar EMG sensors 104 detects muscle activity of muscles connected to the pelvic floor muscles of the body of the patient. An EMG amplifier 105 is in communication with the EMG probe 101 or the EMG sensor set 103. The EMG amplifier 105 includes a plurality of input channels 106. Each input channel 106 receives data of muscle activity in the pelvic floor muscles or the muscles connected to the pelvic floor muscles from the EMG probe 101 or the EMG sensor set 103. A computer 107 is in communication with the EMG amplifier 105. The computer 107 includes a processor and a memory. A more detailed description of an exemplary computer is provided below. The memory stores computer instructions configured to be executed by the processor. The computer 107 instructions instruct the processor to perform muscle network analysis using the data of muscle activity in the pelvic floor muscles or the muscles connected to the pelvic floor muscles. A treatment regimen for CPP in the patient is recommended based on the muscle network analysis.

The electrodes 102 of the EMG probe 101 are positioned internally (e.g., vaginally), and the bipolar electrodes 104 of the EMG sensor set 103 are positioned externally (e.g., about anterior and posterior regions of the patient). The terms "electrode" and "sensor" may be used interchangeably herein.

The EMG probe 101 includes sensor bands 108. Each of the senor bands 108 includes spaced apart sensors arranged circumferentially around the EMG probe 101. The EMG probe 101 includes at least 36 electrodes (e.g., 3 bands of 12 spaced apart electrodes evenly spaced circumferentially about the EMG probe 101, and each of the 3 bands spaced apart from each other) configured to detect pelvic floor muscle activity. The EMG sensor set 103 includes at least 20 bipolar EMG sensors 104 configured to detect muscle activity in hip muscles, leg muscles, back muscles, or abdominal muscles. The input channels 106 of the EMG amplifier 105 include at least 36 input channels in respective communication with the at least 36 electrodes configured to detect pelvic muscle activity. The input channels 106 of the EMG amplifier 105 include at least 20 input channels in respective communication with the at least 20 bipolar EMG sensors 104 configured to detect muscle activity in hip muscles, leg muscles, back muscles, or abdominal muscles.

A fin 109 is positioned at a proximal end portion of the EMG probe 101 opposite a distal end of the EMG probe 101. The fin 109 is aligned with a sensor of the spaced apart sensors 102 to determine a directional orientation of the sensor with respect to the body of the patient. Thus, the fin 109 may be employed to identify positioning of the EMG probe 101 within a patient, such that a particular electrode 102 is adjacent a desired pelvic muscle or pelvic muscle region.

The EMG probe 101, EMG sensory array 103, and the EMG amplifier 105 may communicate wirelessly with each other (e.g., via wireless transmitters/receivers 110, 111, 112). Additionally, a wired or wireless connection may connect the EMG amplifier 105 with a computer (e.g., 107), as described herein. For example, a first wireless module 110 is connected to the EMG probe 101. A second wireless module 111 is connected to the EMG sensor set 103. A third wireless module 112 is connected to the EMG amplifier 105. Each of the first, second, and third wireless modules 110, 111, 112 is configured to transmit or receive the data of muscle activity in the pelvic floor muscles or the muscles connected to the pelvic floor muscles.

In an aspect of the present disclosure, the recommended treatment regimen for CPP is a physical therapy (PT) treatment. The PT treatment includes myofascial therapy or movement training.

In an aspect of the present disclosure, an inter-muscle coherence analysis is performed (see, e.g., FIGS. 4A and 4C) using the data of muscle activity in the pelvic floor muscles or the muscles connected to the pelvic floor muscles of the body of the patient.

In an aspect of the present disclosure, the data of muscle activity in the pelvic floor muscles or the muscles connected to the pelvic floor muscles is received in the EMG amplifier at substantially the same time that the muscle network analysis is performed.

As an example, the muscle network analysis is performed in real-time while a PT treatment is performed on the patient. The computer instructions instruct the processor to perform a second muscle network analysis during the PT treatment and recommend a second treatment regimen for CPP in the patient based on the second muscle network analysis. Muscle network analysis and/or inter-muscle coherence analysis may be performed (e.g., in real-time) at each of a series of PT sessions to show treatment progress, and to identify adjustments to improve the treatment regimen.

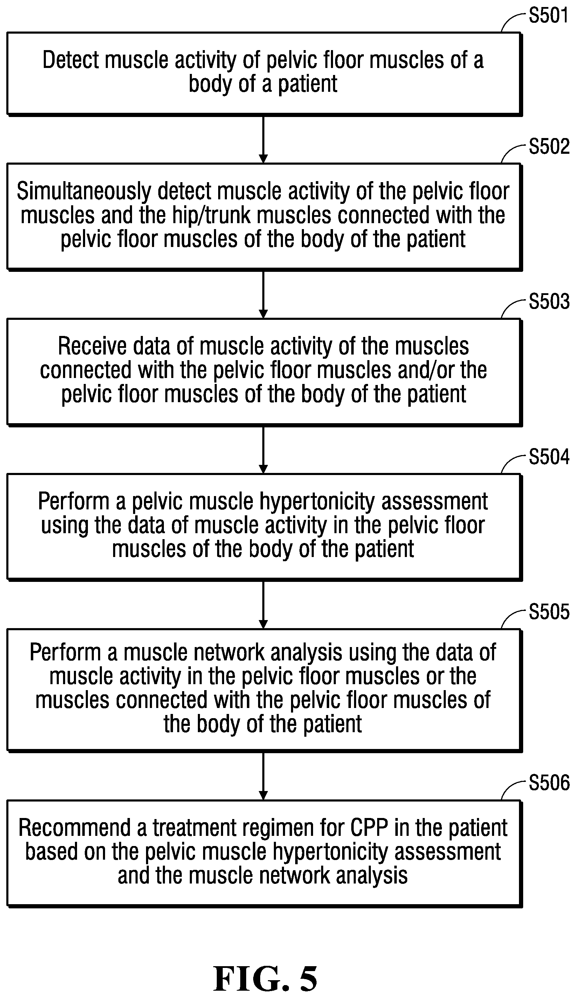

FIG. 5 is a block diagram of a method for determining an individualized treatment regimen for CPP according to aspects of the present disclosure.

Referring to FIG. 5, a method for determining a treatment for CPP includes detecting, by the EMG probe, muscle activity of pelvic floor muscles of a body of a patient (S501). The method includes detecting, by the EMG sensor set, muscle activity of muscles connected to the pelvic floor muscles of the body of the patient (S502). The method includes receiving, at the EMG amplifier, data of muscle activity in the pelvic floor muscles or the muscles connected to the pelvic floor muscles of the body of the patient from the EMG probe or the EMG sensor set (S503). The processor of the computer performs a pelvic muscle hypertonicity assessment using the data of muscle activity in the pelvic floor muscles of the body of the patient (S504). The processor of the computer performs a muscle network analysis using the data of muscle activity in the pelvic floor muscles or the muscles connected to the pelvic floor muscles of the body of the patient (S505). A treatment regimen for CPP in the patient is recommended based on the muscle network analysis (S506).

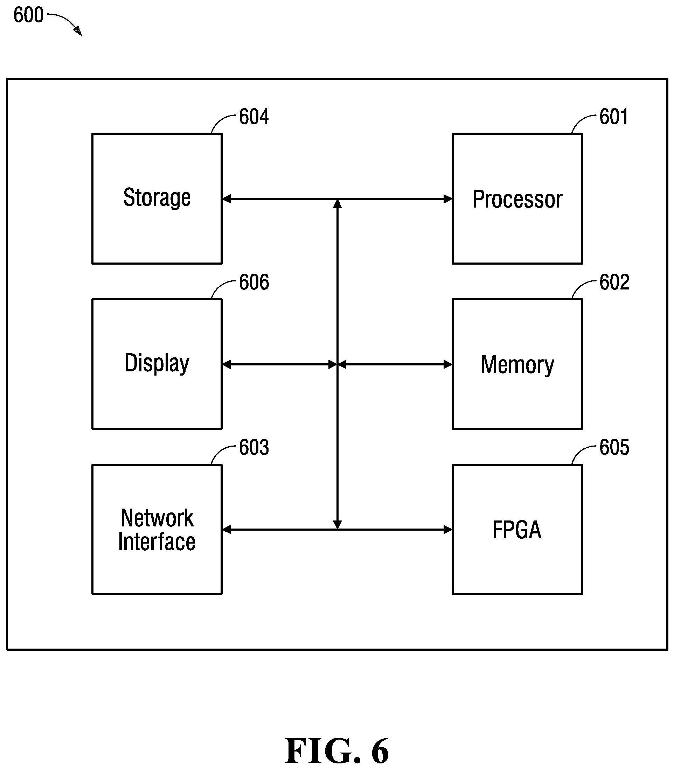

FIG. 6 is a block diagram of an exemplary computer of the system of FIG. 1 according to aspects of the present disclosure.

Referring to FIG. 6, the computer 600 may include a processor 601 connected to a computer-readable storage medium or a memory 602 which may be a volatile type of memory, e.g., RAM, or a non-volatile type memory, e.g., flash media, disk media, etc. The processor 601 may be another type of processor such as, without limitation, a digital signal processor, a microprocessor, an ASIC, a graphics processing unit (GPU), field-programmable gate array (FPGA), or a central processing unit (CPU). The computer 600 may include a display 606.

In some aspects of the disclosure, the memory 602 can be random access memory, read-only memory, magnetic disk memory, solid state memory, optical disc memory, and/or another type of memory. The memory 602 can communicate with the processor 601 through communication buses of a circuit board and/or through communication cables such as serial ATA cables or other types of cables. The memory 602 includes computer-readable instructions that are executable by the processor 601 to operate the control unit. The computer 600 may include a network interface 603 to communicate with other computers or a server. A storage device 604 may be used for storing data. The computer 600 may include one or more FPGAs 605. The FPGA 605 may be used for executing various machine learning algorithms.

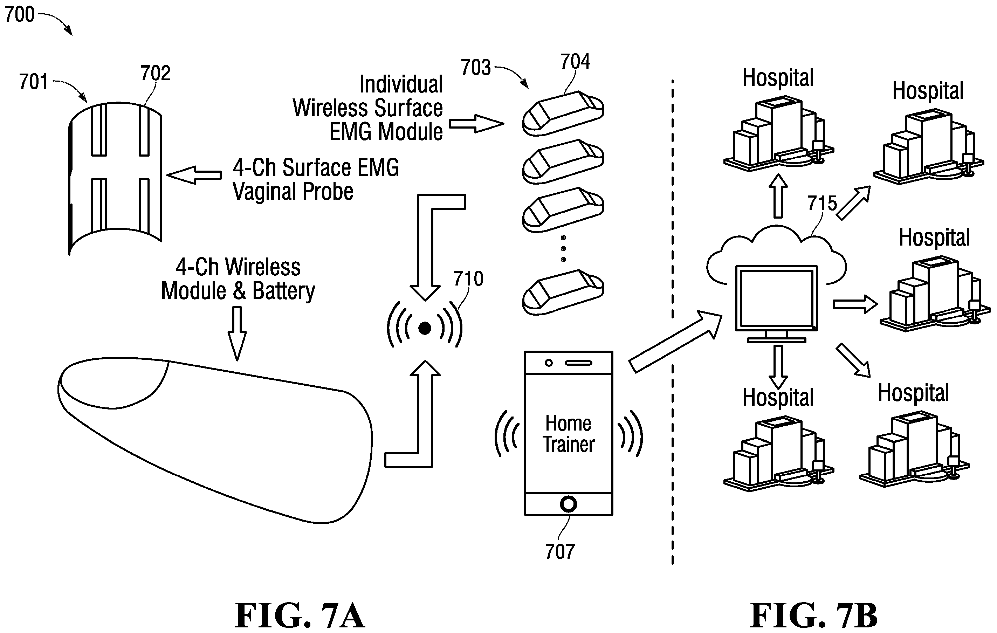

FIGS. 7A and 7B illustrate a smartphone-based system for CPP patient phenotyping and personalized physical therapy system for home use (Fig 7A). The system communicates with healthcare providers for training progress monitoring and reporting (Fig 7B).

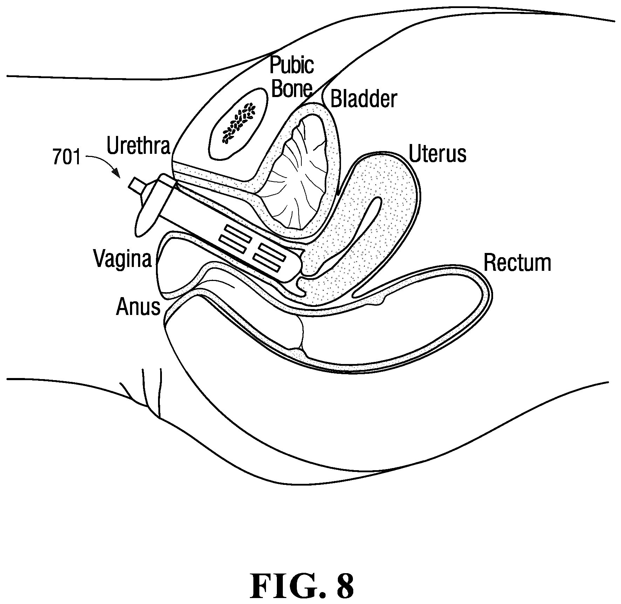

FIG. 8 shows a schematic diagram illustrating the use of the disclosed vaginal surface EMG probe with eight surface EMG sensors for wireless EMG recordings from the pelvic floor muscles.

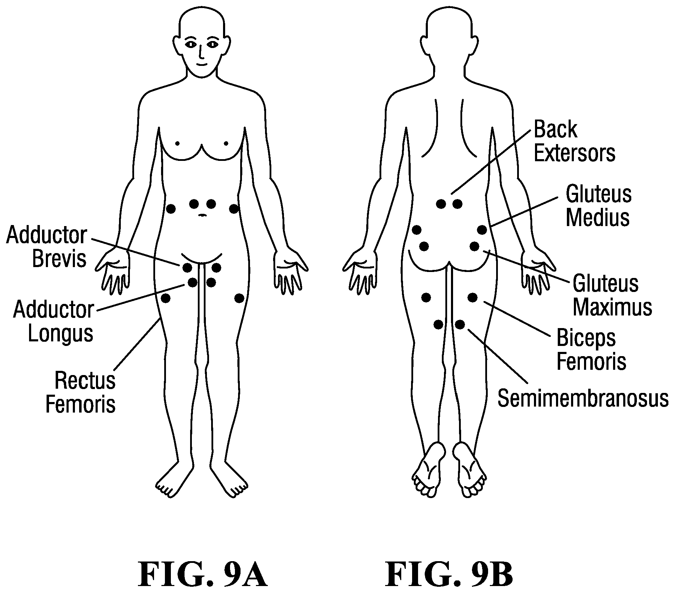

FIGS. 9A and 9B show a schematic diagram illustrating the use of disclosed wireless bipolar surface EMG sensors for EMG recordings from the hip muscles, leg muscles, back muscles, and abdominal muscles. Twenty muscles of interests are marked in the figure. The disclosed apparatus allows for reliably and quantitatively assessing muscle activation pattern for individual muscles and inter-muscle interaction pattern for muscle pairs involved in the PFM-Hip-Trunk muscle network in real time and wirelessly. All these 20 channels can be used for diagnosis and patient phenotyping purposes. Once the PFM to Hip/Trunk muscles pairs with abnormal connection strength are identified, wireless bipolar surface EMG sensors are used for the Hip/Trunk muscles with abnormal connection strength for personalized physical therapeutic training.

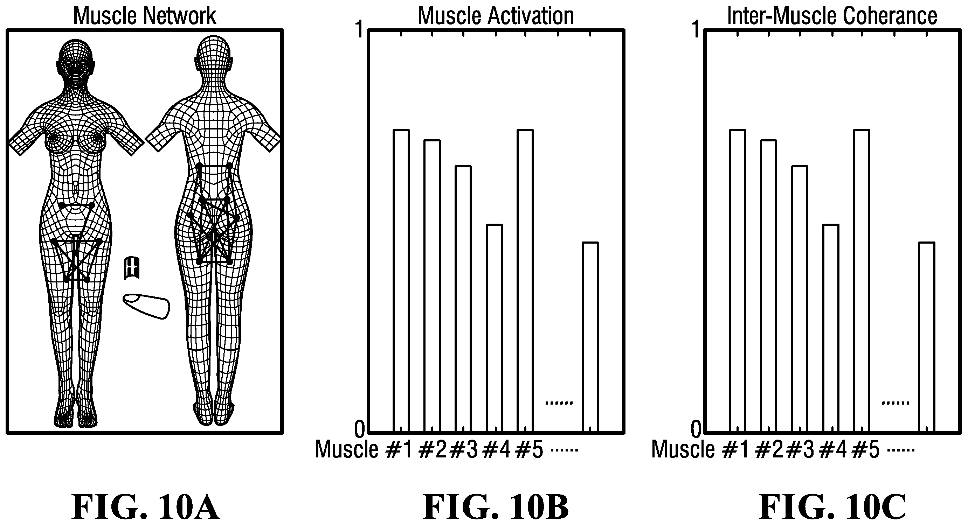

FIGS. 10A, 10B, and 10C show a schematic diagram illustrating the muscle activation pattern for individual muscles and inter-muscle interaction pattern for muscle pairs involved in the PFM-Hip-Trunk muscle network, which change in real time and are used to phenotype patients and guide adaptive and personalized physical training (e.g., in a clinic).

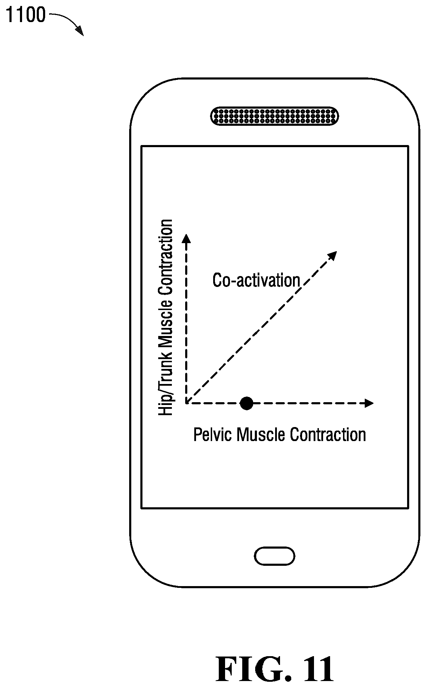

FIG. 11 shows a schematic diagram illustrating personalized physical training using a smartphone, which is connected to the wireless surface EMG system as shown in FIGS. 7A and 7B, and to the healthcare system via the cloud. The dynamic training plot on the screen of the smartphone indicates whether the training which the patient is performing is correctly breaking the abnormal connections (the ball will move horizontally or vertically) or incorrectly worsening the abnormal connections (the ball will move along the 45-digree diagonal line).

Referring to FIGS. 7A to 11, a system 700 for monitoring a treatment for CPP includes an EMG sensor set 703 including a plurality of surface EMG sensors 704 configured to capture data of muscle activity in hip muscles, leg muscles, back muscles, or abdominal muscles of a patient performing a physical therapy treatment regimen for CPP. The physical therapy treatment regimen for CPP is based on abnormal Pelvic Floor Muscle (PFM) to Hip/Trunk muscle connections. A computer (e.g., a computer of a smartphone or tablet computer 707) is in communication with the EMG sensor set 703. An exemplary computer 600 is described in more detail with reference to FIG. 6. The computer is configured to receive the captured data from the EMG sensor set 703. The computer includes a processor and a memory. The memory stores computer instructions configured to be executed by the processor. The computer instructions instruct the processor to perform a muscle activation pattern analysis and an inter-muscle interaction pattern analysis using the captured data from the EMG sensor set 703, and determine if a modification is needed to the physical therapy treatment regimen based at least on the muscle activation pattern analysis or the inter-muscle interaction pattern analysis.

The system 700 may include a wireless module 710 in communication with a cloud-based server 715. The wireless module 710 is configured to transmit the captured data from the EMG sensor set 703 or the modification to the physical therapy treatment regimen for CPP to the cloud-based server 715 for communication with a healthcare provider.

In an aspect of the present disclosure, the surface EMG sensors 704 of the plurality of surface EMG sensors are wireless sensors configured to connect wirelessly with the computer.

With ongoing reference to FIGS. 7A to 11, a smartphone-based system for home use is employed to assess the PFM-Hip-Trunk muscle network using wireless multi-channel surface EMG recording including the vaginal surface EMG recording (see, e.g., EMG vaginal probe 701) and up to 20 bipolar surface EMG recordings from the hip muscles, leg muscles, back muscles, and abdominal muscles which are functionally connected to PFM. The system described with reference to FIGS. 7A to 11 may be embodied in a self-contained apparatus including an onboard computer and a display (see, e.g., display 606 of computer 600).

According to an aspect of the present disclosure, muscle activation patterns for individual muscles and inter-muscle interaction pattern for muscle pairs involved in the PFM-Hip-Trunk muscle network are objectively and quantitatively assessed in real time, from multi-channel surface EMG recordings via a smartphone-based system.

Phenotyping CPP patients for appropriate physical therapy intervention is performed by reliably and quantitatively assessing muscle activation patterns of individual muscles and inter-muscle interaction pattern of muscle pairs involved in the PFM-Hip-Trunk muscle network, via the smartphone-based system and methods described herein.

The systems and methods described herein are employed to offer adaptive and personalized intervention in CPP management by adaptively modifying physical therapy protocol during the multi-session training, guided by dynamic alterations of muscle activation patterns and inter-muscle interaction patterns of the PFM-Hip-Trunk muscle network. The systems and methods described herein, including all captured EMG data, is performed while protecting patients' privacy.

The systems and methods described herein provide a solution for adaptive and personalized physical therapy training at home for the optimized treatment outcome in CPP management, including communicating with healthcare providers via a cloud platform for training progress monitoring and reporting.

The PFM-Hip-Trunk muscle network consists of over 20 muscle groups including the pelvic floor muscles, hip muscles, leg muscles, back muscles and abdominal muscles which can contribute to chronic pelvic pain. The current standard treatment, myofascial physical therapy (MPT) treats specifically the pelvic muscles, and aims to mobilize soft tissue via manual massage, relax the muscle via dilation, or improve muscle coordination with muscle retraining of the pelvic muscles to resolve somatic abnormalities causing pelvic floor pain. However, MPT does not address posture and movement impairments of the trunk and hip, which are associated with the presence of pelvic pain. Complementary to myofascial therapy, movement physical therapy is a treatment philosophy that aims to correct postural dysfunction and correct aberrant movement patterns that may cause pelvic pain. Pelvic floor pain is intrinsically a multifactorial dysfunction that can be attributed to postural issues, myofascial trigger points, peripheral sensitization, and abnormal muscle tone. The systems and methods described herein allow for 1) phenotyping patients for appropriate physical therapy intervention, and 2) personalizing physical therapeutic protocol.

The systems and methods described herein provide 1) an intra-vaginal high-density surface electromyography technique to reliably and quantitatively assess pelvic floor muscle hypertonicity; and 2) a muscle network analysis method to reveal the inter-muscle coherence pattern alterations representing the neural drive from the central nervous system. The systems and methods described herein provide solutions for optimizing CPP management via 1) phenotyping patients for appropriate interventions (myofascial therapy or movement training) and 2) personalizing physical therapy intervention guided using dynamic intermuscular interaction pattern from real-time muscle network analysis based on multi-channel surface EMG signals of the PFM-Hip-Trunk muscle network.

The devices, systems and methods described herein provide a smartphone-based system for home use (home trainer) for optimizing CPP management via 1) phenotyping patients for appropriate interventions (myofascial therapy or movement training) and 2) personalizing physical therapy intervention guided using dynamic intermuscular interaction pattern from real-time muscle network analysis based on multi-channel surface EMG signals of the pelvic floor, hip, leg, back and abdominal muscles. The system described herein includes a portable and low-cost apparatus for multi-channel surface EMG signal acquisition from the pelvic floor muscles (PFM), hip muscles, back muscles, and abdominal muscles, a real-time muscle network analysis method, and a smartphone-based platform which allows for communicating with healthcare providers for progress monitoring and reporting.

According to an aspect of the disclosure, the apparatus includes, for example, wireless vaginal surface EMG sensors 702 over a vaginal probe 701 for multi-channel surface EMG sensing from PFM. The apparatus includes up to 20 wireless surface EMG sensors 7004 sensing from the hip abductors, hip adductors, rectus femoris, Gluteus, back extensors, abdominal muscles, and semimembranosus and biceps femoris. The apparatus includes a wireless data collection module 710 and a smartphone application (see, e.g., smartphone application 1100 in FIG. 11) which can run on smartphones 707 to offer patient phenotyping and personalized training functions. A cloud-based platform 715 communicates with physicians and/or physical therapists (or other healthcare providers) for progress monitoring and reporting.

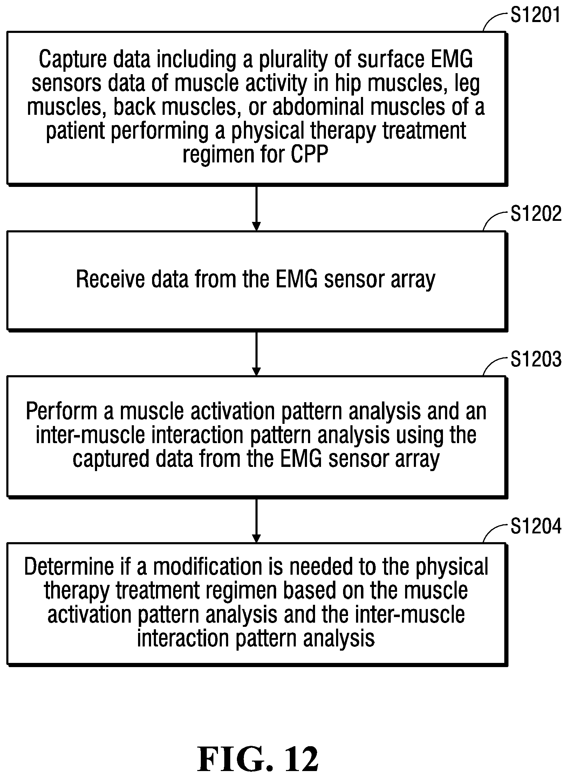

Referring to FIG. 12, a method of monitoring a treatment for CPP includes capturing data, by an EMG sensor set including a plurality of surface EMG sensors data of muscle activity in hip muscles, leg muscles, back muscles, or abdominal muscles of a patient performing a physical therapy treatment regimen for CPP (S1201). The physical therapy treatment regimen for CPP is based on abnormal Pelvic Floor Muscle (PFM) to Hip/Trunk muscle connections. The method includes receiving, by a computer, data from the EMG sensor set (S1202). The method includes performing, by the computer, a muscle activation pattern analysis and an inter-muscle interaction pattern analysis using the captured data from the EMG sensor set (S1203). The method includes determining, by the computer, if a modification is needed to the physical therapy treatment regimen based on the muscle activation pattern analysis and the inter-muscle interaction pattern analysis (S1204).

In an aspect of the present disclosure, the method includes determining a modification to an at-home physical therapy regimen including a plurality of physical therapy sessions. For example, a different combination of at-home physical therapy exercises may be suggested, or a duration or schedule of physical therapy exercises may be modified.

Imaging modalities such as ultrasound and MRI can detect anatomical abnormalities. Still, they cannot assess the functional status and innervation of muscles, which may be critical in the presence of pain syndromes. Pain arising from PFMs with myofascial trigger points is believed to result from an excessive release of acetylcholine from NMJs after chronic muscle hypercontraction. Intramuscular EMG can detect abnormally increased neuromuscular activity, but it is painful, and spatially limited to a small uptake area of the needle electrode. The digital pelvic exam provides subjective information regarding PFH. Objective and quantitative measures of PFM function in IC/BPS patients are lacking, which would otherwise help elucidate the contribution of pelvic floor dysfunction to the pathophysiology of IC/BPS and how IC/BPS affects the PFM.

Clinical management of PFH involves the retraining and rehabilitation of the dysfunctional muscles, often through behavioral and physical therapy, oral medications, neuromodulation, and trigger point injections. Despite these methods, the management of PFH remains challenging and sometimes inefficient due to its multifactorial nature. Currently, botulinum neurotoxin (BoNT) is receiving growing interest in relieving PFH and myofascial pain, with superior performance compared to conventional therapies. The symptom relief is attributed to the blockage of acetylcholine neurotransmitters release at the neuromuscular junction (NMJ) that are involved in muscle contraction, nociceptive signaling, and central sensitization.

Despite its proven potency and relative safety in the management of hypertonicity and myofascial pain, BoNT therapy is expensive and can cause dose-dependent adverse effects including muscle atrophy and loss of contractile tissue, pain at the injection site, weakening and atrophy of off-target muscles, systemic toxicity, development of drug resistance, and pelvic disorders such as urinary incontinence, urinary retention, worsening constipation, and fecal incontinence. Moreover, considerable variation of treatment outcome has been reported, along with a non-responding rate of up to 38%. These issues may be attributed to the varied dosages and non-targeted injections. Studies have demonstrated that increasing the injection distance by 1cm from the NMJ of muscles reduced the effect of botulinum toxin injection by 46%. These complications and variable treatment efficacy have reinforced the necessity of precise and reliable injection techniques to minimize the required dose of toxin and therapy cost while maintaining stable, optimized treatment effectiveness.

There is no technique currently available for mapping NMJ distributions over the pelvic floor muscles to guide botulinum toxin injections in specific patients, because of the complexity of the pelvic anatomy. Considerable variation of treatment outcome has been reported, along with a non-responding rate of up to 38%. These issues may be attributed to the varied dosages and non-targeted injections. Studies have demonstrated that increasing the injection distance by 1cm from the NMJ of muscles reduced the effect of botulinum toxin injections by 46%. These complications and variable treatment efficacy have reinforced the necessity of precise and reliable injection techniques to minimize the required dose of toxin and therapy cost while maintaining stable, optimized treatment effectiveness.

The present disclosure provides a novel neuromuscular junction mapping technique by combining the muscle activity imaging and surface EMG decomposition methods to map the NMJ locations over the pelvic floor muscles from a vaginal high-density surface EMG recordings. The map of neuromuscular junctions in hypertonic pelvic muscles can be used to precisely guide the botulinum toxin injection precisely into the NMJs of the pelvic muscles for best treatment outcome.

Quantitatively assessing pelvic floor hypertonicity with high spatial resolution using intra-vaginal HD-sEMG is technically innovative. The deep pelvic floor muscles, including the levator ani muscle are located several centimeters away from the superficial perineum, making direct recording from the skin surface impossible. The disclosed method employs an intra-vaginal high-density (64-channel) surface EMG probe to evaluate the PFM hypertonicity using the abundant spatiotemporal information captured. The disclosed method provides for objectively assessing and phenotyping neuromuscular function of the pelvic floor.

The disclosed method provides for non-invasively imaging pelvic muscle NMJ distributions in vivo. It is very challenging to localize pelvic muscle NMJs primarily because of the complex pelvic anatomy, impeding the direct access of multi-channel surface EMG sensors. By providing pelvic muscle NMJ distribution information and suppressing pelvic muscle crosstalk, for the first time, the NMJ imaging technique will provide critical information for phenotyping axonal or central neurodegeneration, monitoring neuromuscular remodeling, and guiding the precision injection of BoNT for optimal treatment outcome. This is especially the case in patients with neuromuscular disorders, where neuromuscular deficits can alter the distribution of NMJs.

The disclosed method includes using HD-sEMG to guide the BoNT therapy in managing PFH. Current BoNT injection protocol is based on a fixed injection template or manual palpation; as such, the treatment efficacy is largely experience-dependent. HD-sEMG has been proven to be the only non-invasive method to characterize muscle innervations in vivo. Such information will assist in better clinical decision making by selecting personalized injection sites and doses to the pelvic floor to achieve the best treatment outcome.

The design of the apparatus, of the present disclosure presents a solution for personalized precision vaginal botulinum toxin injection with a wireless vaginal surface EMG probe to protect patients' privacy.

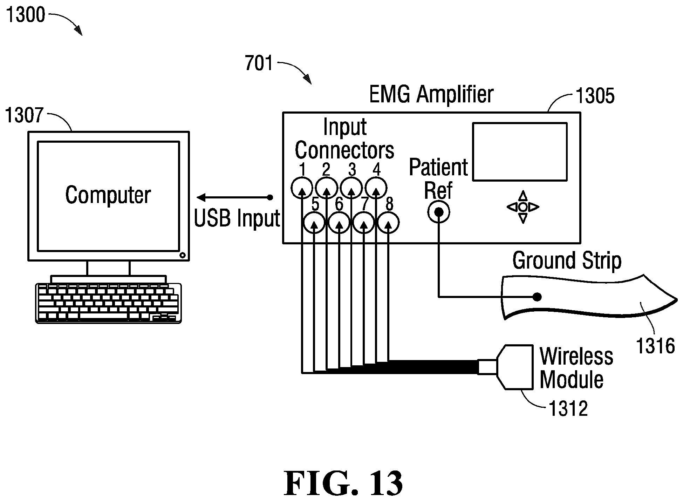

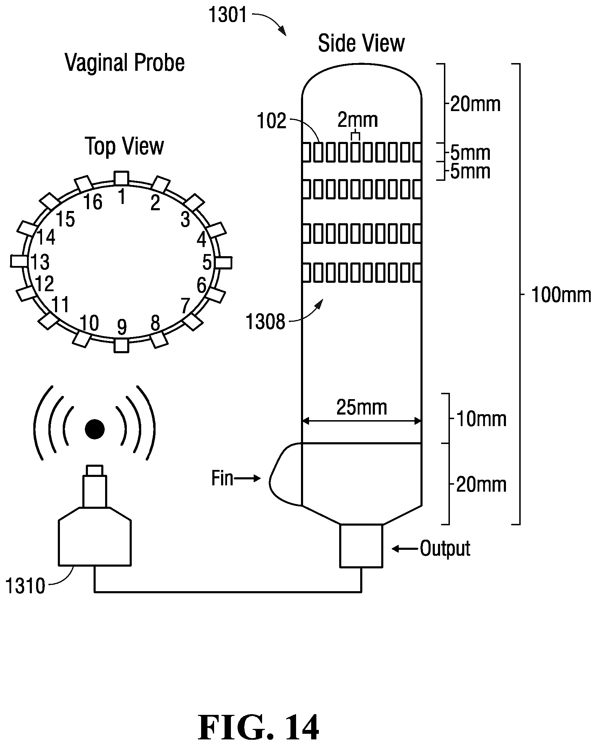

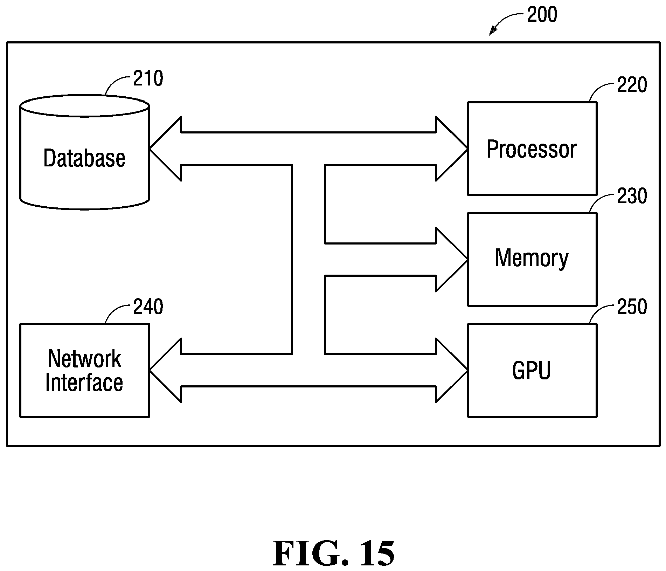

Referring to FIG. 13, a vaginal high-density surface electromyography (HD-sEMG) evaluation apparatus of system 1300 is shown, in accordance with aspects of the present disclosure. The HD-sEMG evaluation apparatus generally includes a controller 200 (e.g., a computing device; see, e.g., FIG. 15 for a more detailed description of an exemplary computing device), an EMG amplifier 1305, a ground strip 1316, a wireless module 1312, and a probe 1301 (e.g., an intra-vaginal probe 1301 shown in FIG. 14). The EMG amplifier 1305 is configured to amplify general and skeletal muscle electrical activity. The controller 200 may be embodied in computer 1307.

Referring to FIG. 14, the intra-vaginal probe 1301 of the HD-sEMG evaluation system/apparatus of FIG. 13 is shown, in accordance with aspects of the present disclosure. The vaginal probe 1301 may include a surface electrode grid 1308 including a plurality of electrodes/sensors 1302, and a wireless module 1310 configured to wirelessly communicate with the wireless module 1312 of the HD-sEMG evaluation apparatus.

Referring now to FIG. 15, there is shown an illustration of exemplary components in the controller 200 of FIG. 13, in accordance with aspects of the present disclosure. The controller 200 includes, for example, a database 210, one or more processors 220, at least one memory 230, a network interface 240, a general processing unit (GPU), and a database 210.

The database 210 can be located in a storage. The term "storage" may refer to any device or material from which information may be capable of being accessed, reproduced, and/or held in an electromagnetic or optical form for access by a computer processor. A storage may be, for example, volatile memory such as RAM, non-volatile memory, which permanently hold digital data until purposely erased, such as flash memory, magnetic devices such as hard disk drives, and optical media such as a CD, DVD, Blu-ray disc, cloud storage, or the like.

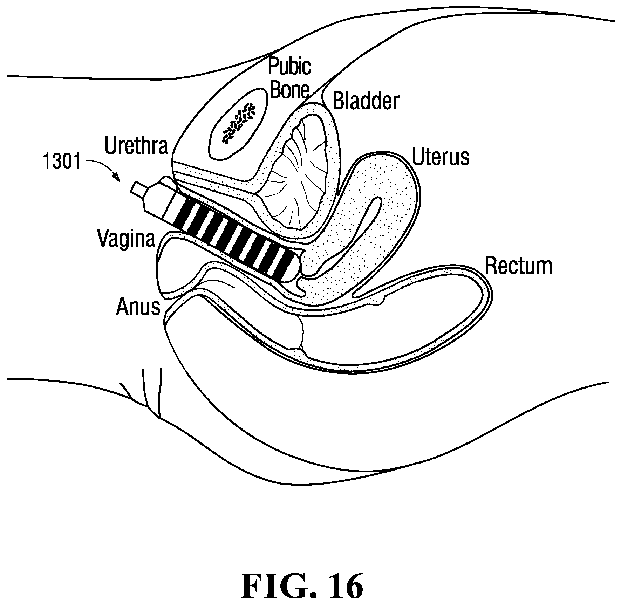

Referring to FIG. 16, is a side view of the vaginal probe of FIG. 14 introduced into a vaginal space.

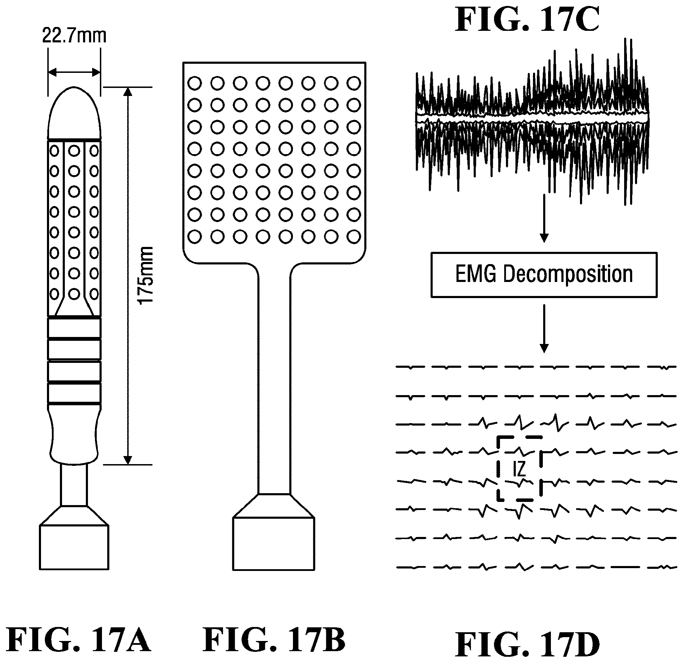

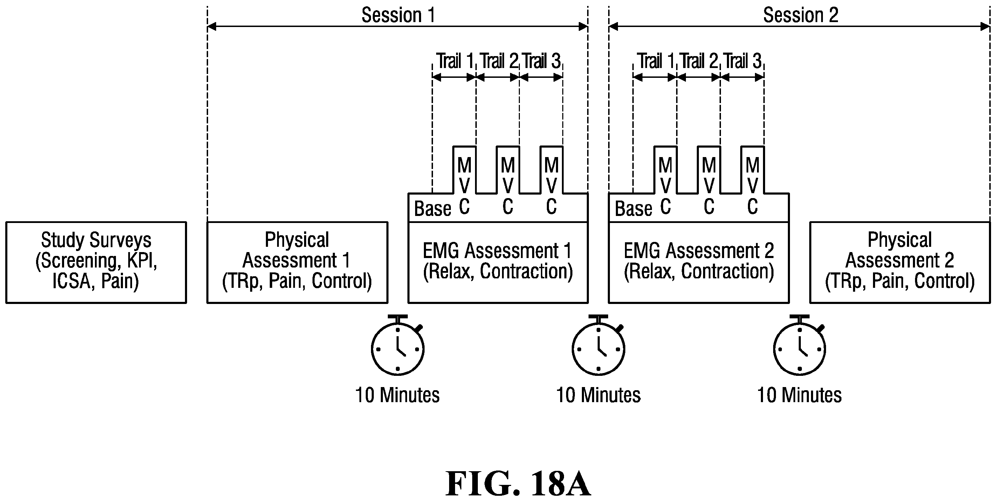

Referring to FIGS. 17A-D and 18A, HD-sEMG acquisition commenced after the first pelvic assessment utilizing the 64-channel intra-vaginal HD-sEMG probe (FIG. 17A). For example, the intra-vaginal HD-sEMG probe may include a 64 circular surface electrode with a diameter of about 4.0 mm, and inter-electrode spacing (center to center) of about 8.8 mm both longitudinally and circumferentially. The intra-vaginal HD-sEMG probe may include a vaginal probe covered by an 8x8 surface electrode grid (FIG. 17B). The HD-sEMG probe may be lubricated with conductive gel and introduced into the vaginal space by a clinician (e.g., a urologist). Probe orientation may be standardized between sessions by aligning the electrode gap along the probe's dorsal surface. A ground electrode may be attached to the right wrist of the patient. An adhesive reference electrode may be attached to the right thigh. HD-sEMG signals may be recorded at, for example, 2048Hz with an amplifier, as shown in FIG. 17C. Approximately a 60-second resting period may be allowed for electrode-mucosa contact stabilization, once stabilization was assured, an additional 10-second resting period was allowed (resting trial 1) followed by a 10-second maximum voluntary contraction (MVC) (MVC trial 1). The resting and MVC trials may be repeated alternately to give a total of 3 trials for each in Session 1, as shown in FIG. 18A. The probe may then be removed, and a rest period (e.g., about 10-minutes) may be given. The process may then be repeated in Session 2. In the disclosed method the resting HD-sEMG data and MVC HD-sEMG data may be used to calculate a pelvic muscle hypertonicity index (e.g., a resting RMS ratio).

HD-sEMG signals may be band-pass filtered between 10Hz and 500Hz via a filter (e.g., a second-order Butterworth filter). Mains interference may be attenuated with a 60Hz notch filter (e.g., a Butterworth notch filter). Differential EMG traces for each channel may be segmented into trials based on the paradigm described in FIG. 18A-B, and the root mean square (RMS) values may be calculated channel-wise in half-second intervals for each resting trial and averaged. The resting RMS ratio may be calculated channel-wise for each trial by normalizing the averaged resting RMS values to the peak RMS reached during the corresponding MVC trial. The resting RMS ratios then may be averaged across the 3 resting trials in each session for each channel to form a 64-channel resting RMS ratio map for each participant.

Filtered HD-sEMG signals may be acquired during rest and MVC may be decomposed using the k-means clustering convolution kernel compensation algorithm, into motor unit action potentials (MUAP). Motor Unit Action Potentials. MUAPs may be produced by the summation of electrical potentials of individual muscle fibers innervated by the same motor neuron and are activated by voluntary muscle contractions. One of skill in the art would know what k-means clustering convolution kernel compensation algorithm is and how to implement it. The innervation zone of each motor unit, which indicates the neuromuscular junction, can be identified from a bipolar map of the 64-channel MUAPs by checking the phase reversion of the propagating signals along muscle fibers, as shown in FIG. 17D.

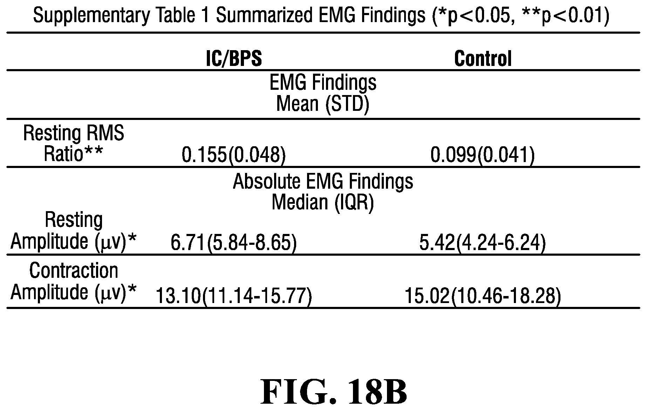

With reference to FIG. 18B a table of example EMG findings is shown, in accordance with aspects of the present disclosure. For example, women with IC/BPS reported higher IC Symptom Index, IC Problem Index, and numeric ratings of pain compared to controls (p<0.001). All women with IC/BPS reported pelvic floor tenderness in at least 1 muscle upon palpation. No controls reported tenderness upon palpation. Categorical variables were compared via a chi-squared test for independence. Myofascial trigger points were present in 86.7% of IC/BPS and 13.3% of controls during the first pelvic exam (p<0.01). PFM control was assessed during the digital pelvic exam. 80% of IC/BPS demonstrated shortened PFM alignment at rest compared to 13.4% of controls (p<0.01). 80% of IC/BPS and 100% of controls could relax the PFM (p=0.224). 40% of IC/BPS and 73.3% of controls could lower the PFM (p=0.141).

HD-sEMG signals were successfully acquired from all participants. Average resting RMS was compared with Mann-Whitney U-tests and was found to be significantly increased in the IC/BPS group (p=0.0344), but not average contraction RMS amplitude (p=0.91). The resting RMS ratio was calculated by normalizing the resting EMG RMS to the corresponding peak MVC amplitude. Interclass correlation coefficient (ICC) between sessions was assessed for resting RMS ratios in controls, and the high ICC value of 0.77 demonstrate the high reliability of resting RMS ratio calculation from intra-vaginal HD-sEMG recordings.

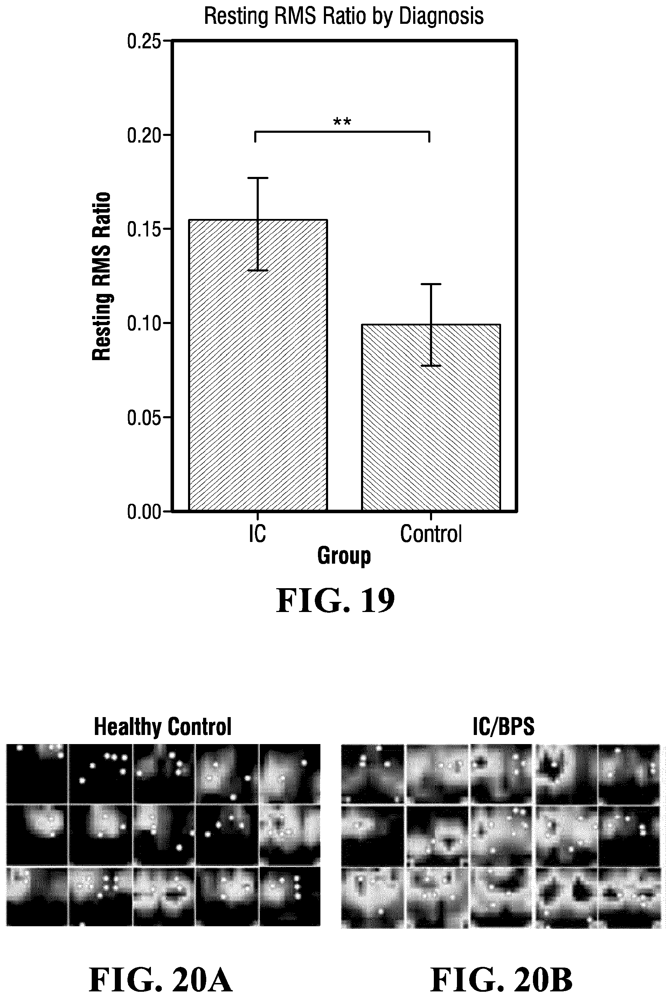

FIG. 19 shows the average resting RMS ratio for both groups. Average resting RMS ratio was compared between diagnosis groups with a student's t-test, and was found to be significantly higher in the IC/BPS group compared to controls (p=0.0019), suggesting that women in the IC/BPS group had either increased resting EMG, spatially broad region of increased resting EMG, or decreased peak amplitude during MVC, or both. The spatial distribution of the single-channel resting RMS ratios for all participants is presented in FIGS. 20A-B.

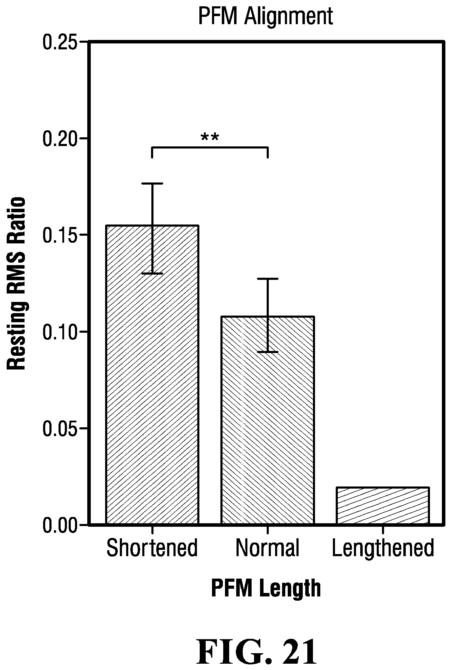

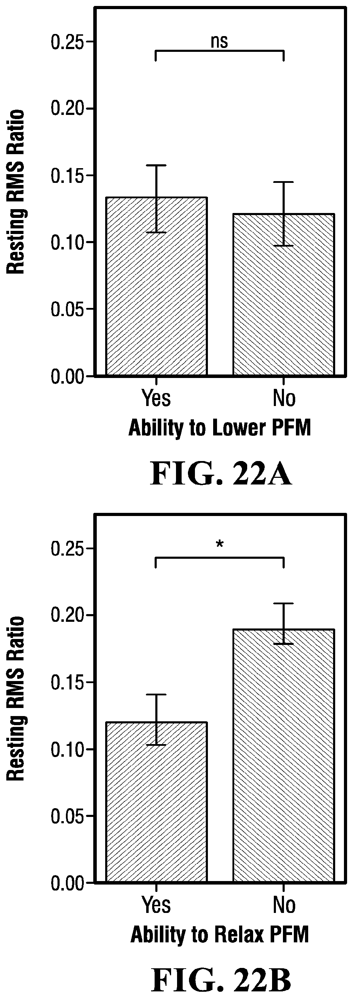

As shown in FIGS. 21 and 22A-B, resting RMS ratio was compared between PFM functional evaluation groups with a student's t-test. Women from both groups with normal, shortened or lengthened PFMs were grouped, and a significantly increased resting RMS ratio was found in women with a shortened pelvic floor (p=0.0058). There was no significant difference in resting RMS ratio between women who could not lower their PFM versus those who could (p>0.4). Women who could not relax their PFM showed significantly increased resting RMS ratio in comparison to women who could (p=0.028).

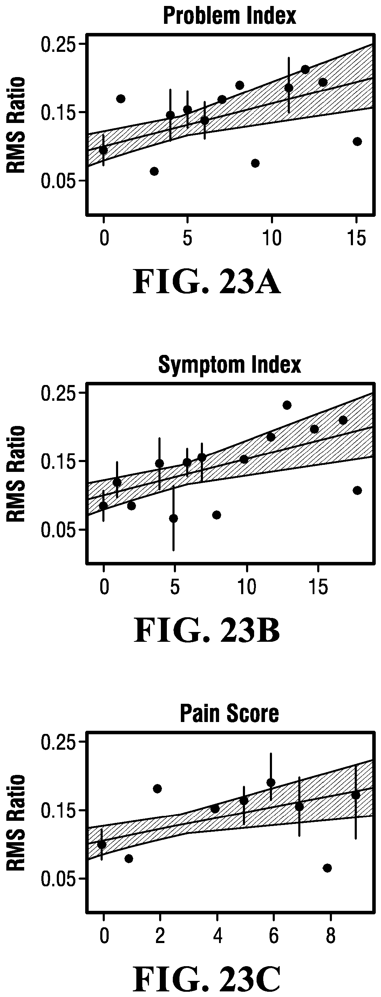

The relationship between resting RMS ratio and self-reported IC Symptom Index, IC Problem Index, and 0-10 pain scores was assessed by Ordinary Least Squares regression, as shown in FIGS. 23A-C. A significant linear relationship was found between the resting RMS ration and the patient-reported clinical scores (ICSI, ICPI, Pain) (p<0.003) in all cases.

HD-sEMG has the unique ability to localize motor unit (MU) innervation zones (IZs). By leveraging the abundant spatiotemporal information afforded by HD-sEMG to decompose the EMG into constitutive MUs, MU signal propagation along the muscle fibers innervated by a single motor neuron can be investigated. This technique may be used to study the innervation zones of women with myofascial pain resulting from IC/BPS. Decomposing and localizing innervation zones was successful, and a unique IZ distribution was present across all subjects, shown in FIGS. 20A-B.

OnabotulinumtoxinA (BoNT, Botulinum toxin) is receiving growing interest in relieving PFH and myofascial pain. However, considerable outcome variation has been reported. This variability may be partially attributed non-targeted injections. Critical to maximizing the efficacy of BoNT is injection proximity to the IZ. A future application of the presented technique is IZ targeted PFM BoNT injections. Nesbitt-Hawes et. al proposed a four-dimensional ultrasound-based injection technique, with promising results, which may be further improved upon by including IZ targeted injections. BoNT injections targeting hypertonic PFMs have been investigated under iEMG guidance with promising results. However, iEMG is painful, requiring multiple insertions to locate a hypertonic muscle. The presented HD-sEMG probe requires a single acquisition to generate a high-density map of PFM activity and patient-specific IZ distribution. Future studies may aim to assess therapeutic changes when BoNT is injected towards patient-specific IZ distributions.

The presented HD-sEMG technique provides a method to non-invasively quantify and localize electrical output from the PFMs and found that women with IC/BPS had significantly higher normalized EMG at rest compared to controls. The HD-sEMG technique allows for spatial mappings depicting the electrical output from the muscle at rest, normalized by the peak amplitude reached during MVC, unveiling unique hypertonicity distribution patterns and pelvic floor phenotyping features in women with IC/BPS. Furthermore, the spatiotemporal high resolution provided by the 64-channel electrode grid allows for the decomposition of EMG signals, allowing for the localization of individual IZs. This technique may be useful for targeting BoNT injections to the PFM IZs. In various methods, the BoNT dosage may be determined by the severity assessment.

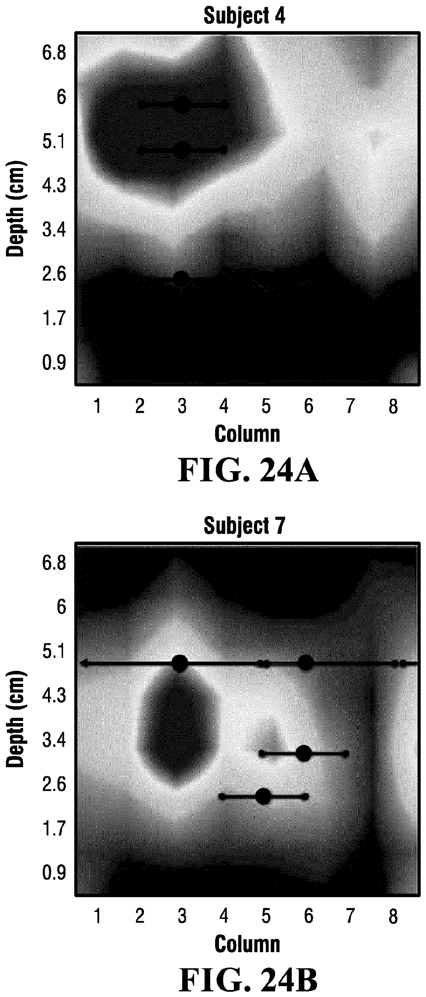

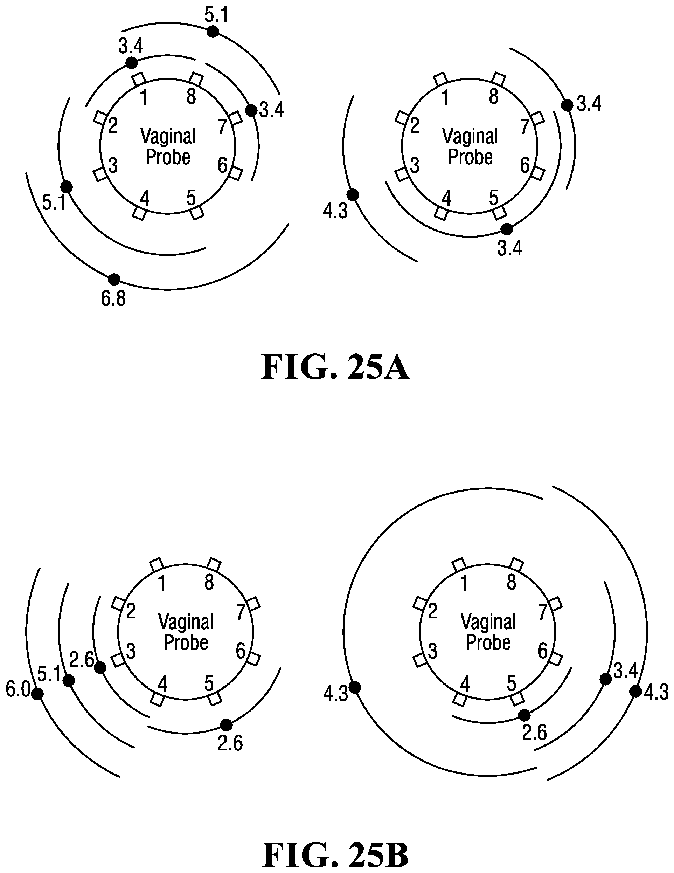

With reference to FIGS. 24A-B, images of high-density EMG in pelvic floor hypertonicity are shown. Another advantage of HD-sEMG based PFM assessment is the ability to discern IZs at a sub-centimeter resolution. IZs are commonly identified using an evenly-spaced linear electrode array that is placed along the muscle fiber direction. A visual based IZ detection technique may be used to compare the locations of IZs to myofascial trigger point locations in the upper trapezius muscle. Trigger points are proximally located to the IZ. As shown in FIGS. 25A-B, an IZ near the hypertonic zone, may be localized for both subjects with hypertonicity directly from the differential surface interference pattern EMG data, suggesting the feasibility of an HD-sEMG guided injection protocol directed to the IZ. External anal sphincter IZ distributions may be assessed by employing visual identification of signal phase inversion. If IZs are unable to be discerned directly from the surface interference pattern, a valid alternative is a decomposition-based IZ detection scheme. However, this method is negatively affected by misalignments between fiber direction and electrode configuration. Linear electrode arrays are also limited by the substantial signal crosstalk, which may confuse IZs with neighboring myoelectric sources. The feasibility of characterizing IZ distribution in the pelvic muscles of healthy participants using pelvic HD-sEMG. HD-sEMG decomposition may be performed to suppress signal crosstalk and generate a clean mapping for the estimation. HD-sEMG decomposition may be employed to generate an IZ distribution of the PFMs. According to the present method, personalized IZ mappings may be successfully generated for each subject. A marked inter-subject variation of the IZ maps was observed, as shown in FIGS 25A-B, which coincides with observations of varied IZ distribution in limb muscles. This finding stresses the importance of a personalized injection strategy to maximize BoNT efficiency.

With continued reference to FIGS. 25A-B, derived axial IZ mappings are shown, which can be used to optimize the treatment efficacy of BoNT for alleviating PFH. The efficacy of BoNT therapy can likely be potentiated by endplate targeted injections achieved by specifying the muscle(s) responsible for PFH, as well as the offending IZ. A 46% reduction in efficacy is noted when BoNT was injected 1 cm away from the IZ, stressing the importance of accurate injection guidance.

Any of the herein described methods, programs, algorithms or codes may be converted to, or expressed in, a programming language or computer program. The terms "programming language" and "computer program," as used herein, each include any language used to specify instructions (i.e., computer instructions) to a processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

It will be understood that various modifications may be made to the aspects and features disclosed herein. Therefore, the above description should not be construed as limiting, but merely as exemplifications of various aspects and features. The invention is as defined in the claims as follows.

## Claims

1. A system (700) for monitoring a treatment regimen for chronic pelvic pain (CPP), comprising:
an EMG sensor set (703) including a plurality of surface EMG sensors (704) configured to capture data of muscle activity in hip muscles, leg muscles, back muscles, or abdominal muscles of a patient performing a physical therapy treatment regimen for CPP, wherein the physical therapy treatment regimen for CPP is based on abnormal Pelvic Floor Muscle (PFM) to Hip/Trunk muscle connections; and
a computer (600, 707) in communication with the EMG sensor set (703) and configured to receive the captured data from the EMG sensor set (703), the computer (600, 707) including a processor and a memory, the memory storing computer instructions configured to be executed by the processor, wherein
the computer instructions configured to instruct the processor to:
perform a muscle activation pattern analysis and an inter-muscle interaction pattern analysis for quantitatively assessing muscle pairs involved in the PFM-Hip-Trunk muscle network using the captured data from the EMG sensor set,
perform a decomposition to suppress muscle crosstalk based on the muscle activation pattern analysis and the inter-muscle interaction pattern analysis,
generate neuromuscular junction mapping information, and
determine if a modification is needed to the physical therapy treatment regimen based on the muscle activation pattern analysis and the neuromuscular junction mapping information.

2. The system (700) of claim 1, wherein the computer (600) is in communication with the EMG sensor set (703) is a smartphone or tablet computer (707).

3. The system (700) of claim 1, further including a wireless module (710) in communication with a cloud-based server (715), the wireless module (710) configured to transmit the captured data from the EMG sensor set (703) or the modification to the physical therapy treatment regimen for CPP to the cloud-based server (715).

4. The system (700) of claim 1, wherein the surface EMG sensors (704) of the plurality of surface EMG sensors (704) are wireless sensors configured to connect wirelessly with the computer (600, 707).

5. The system (700) of claim 1, wherein the physical therapy treatment regimen for CPP includes an at-home physical therapy regimen including a plurality of physical therapy sessions.

6. The system (700) of claim 1, further comprising a display (606) configured to display the modification needed to the physical therapy treatment regimen.

7. The apparatus (700) of claim 1, further including a wireless module (710) in communication with a cloud-based server (715), the wireless module (710) configured to transmit the captured data from the EMG sensor set (703) or the modification to the physical therapy treatment regimen for CPP to the cloud-based server (715) for communication with a healthcare provider.

## Patentansprüche

1. System (700) zur Überwachung eines Behandlungsschemas für chronische Beckenschmerzen (CPP), umfassend:
einen EMG-Sensorsatz (703), enthaltend eine Mehrzahl von Oberflächen-EMG-Sensoren (704), ausgebildet zum Erfassen von Daten von Muskelaktivität in Hüftmuskeln, Beinmuskeln, Rückenmuskeln oder Bauchmuskeln eines Patienten, der ein Physiotherapie-Behandlungsschema für CPP durchführt, wobei das Physiotherapie-Behandlungsschema für CPP auf abnormalen Beckenbodenmuskel- (PFM) zu Hüft-/Rumpfmuskel-Verbindungen basiert; und
einen Computer (600, 707), der mit dem EMG-Sensorsatz (703) in Verbindung steht und ausgebildet ist, um die erfassten Daten von dem EMG-Sensorsatz (703) zu empfangen, wobei der Computer (600, 707) einen Prozessor und einen Speicher enthält, wobei der Speicher Computeranweisungen speichert, ausgebildet, um von dem Prozessor ausgeführt zu werden, wobei
die Computeranweisungen ausgebildet sind, um den Prozessor anzuweisen:
eine Muskelaktivierungsmuster-Analyse und eine Inter-Muskel-Interaktionsmuster-Analyse durchzuführen, um Muskelpaare, die an dem PFM-Hüft-Rumpfmuskel-Netzwerk involviert sind, unter Verwendung der erfassten Daten von dem EMG-Sensorsatz quantitativ zu bewerten,
eine Dekomposition zur Unterdrückung von Muskelübersprechen basierend auf der Muskelaktivierungsmuster-Analyse und der Inter-Muskel-Interaktionsmuster-Analyse durchzuführen,
Erzeugen von Informationen über die neuromuskuläre Verbindung, und
basierend auf der Muskelaktivierungsmuster-Analyse und der Information über die neuromuskuläre Verbindung zu bestimmen, ob eine Modifikation des Physiotherapie-Behandlungsschemas erforderlich ist.

2. System (700) nach Anspruch 1, wobei der mit dem EMG-Sensorsatz (703) in Verbindung stehende Computer (600) ein Smartphone oder Tablet-Computer (707) ist.

3. System (700) nach Anspruch 1, ferner enthaltend ein drahtloses Modul (710) in Kommunikation mit einem Cloud-basierten Server (715), wobei das drahtlose Modul (710) ausgebildet ist, um die erfassten Daten von dem EMG-Sensorsatz (703) oder die Modifikation des Physiotherapie-Behandlungsschemas für CPP an den Cloud-basierten Server (715) zu übertragen.

4. System (700) nach Anspruch 1, wobei die Oberflächen-EMG-Sensoren (704) der Mehrzahl von Oberflächen-EMG-Sensoren (704) drahtlose Sensoren sind, ausgebildet, um sich drahtlos mit dem Computer (600, 707) zu verbinden.

5. System (700) nach Anspruch 1, wobei das Physiotherapie-Behandlungsschema für CPP ein Physiotherapie-Schema für zu Hause enthält, enthaltend eine Mehrzahl von Physiotherapie-Sitzungen.

6. System (700) nach Anspruch 1, ferner umfassend eine Anzeige (606), ausgebildet, um die erforderliche Modifikation des Physiotherapie-Behandlungsschemas anzuzeigen.

7. Vorrichtung (700) nach Anspruch 1, ferner enthaltend ein drahtloses Modul (710) in Kommunikation mit einem Cloud-basierten Server (715), wobei das drahtlose Modul (710) ausgebildet ist, um die erfassten Daten von dem EMG-Sensorsatz (703) oder die Modifikation des Physiotherapie-Behandlungsschemas für CPP an den Cloud-basierten Server (715) zur Kommunikation mit einem Gesundheitsdienstleister zu übertragen.

## Revendications

1. Un système (700) de surveillance d'un régime de traitement de la douleur pelvienne chronique (CPP), comprenant :
un set de capteurs EMG (703) incluant une pluralité de capteurs EMG de surface (704) configurés pour capturer des données d'activité musculaire dans les muscles de la hanche, les muscles des jambes, les muscles du dos ou les muscles abdominaux d'un patient effectuant un régime de traitement de physiothérapie pour CPP, dans lequel le régime de traitement de physiothérapie pour CPP est basé sur des connexions anormales des muscles du plancher pelvien (PFM) aux muscles de la hanche/du tronc ; et
un ordinateur (600, 707) en communication avec le set de capteurs EMG (703) et configuré pour recevoir les données capturées par le set de capteurs EMG (703), l'ordinateur (600, 707) incluant un processeur et une mémoire, la mémoire stockant des instructions d'ordinateur configurées pour être exécutées par le processeur, dans lequel
les instructions d'ordinateur sont configurées pour instruire le processeur à :
effectuer une analyse du modèle d'activation musculaire et une analyse du modèle d'interaction inter-musculaire pour évaluer quantitativement les paires de muscles impliquées dans le réseau musculaire PFM-hanche-tronc en utilisant les données capturées par le set de capteurs EMG,
effectuer une décomposition pour supprimer la diaphonie musculaire sur la base de l'analyse du modèle d'activation musculaire et de l'analyse du modèle d'interaction inter-musculaire,
générer des informations de mappage des jonctions neuromusculaires, et
déterminer si une modification du régime de traitement de physiothérapie est nécessaire sur la base de l'analyse du modèle d'activation musculaire et des informations de mappage des jonctions neuromusculaires.

2. Le système (700) selon la revendication 1, dans lequel l'ordinateur (600) est en communication avec le set de capteurs EMG (703) est un smartphone ou une tablette ordinateur (707).

3. Le système (700) selon la revendication 1, incluant en outre un module sans fil (710) en communication avec un serveur basé dans le nuage (715), le module sans fil (710) configuré pour transmettre les données capturées à partir du set de capteurs EMG (703) ou la modification du régime de traitement de physiothérapie pour le CPP au serveur basé dans le nuage (715).

4. Le système (700) selon la revendication 1, dans lequel les capteurs EMG de surface (704) de la pluralité de capteurs EMG de surface (704) sont des capteurs sans fil configurés pour se connecter sans fil à l'ordinateur (600, 707).

5. Le système (700) selon la revendication 1, dans lequel le régime de traitement de physiothérapie pour le CPP inclut un programme de physiothérapie à domicile incluant une pluralité de séances de physiothérapie.

6. Le système (700) selon la revendication 1, comprenant en outre un écran (606) configuré pour afficher la modification nécessaire au régime de traitement de physiothérapie.

7. Le dispositif (700) selon la revendication 1, incluant en outre un module sans fil (710) en communication avec un serveur basé dans le nuage (715), le module sans fil (710) configuré pour transmettre les données capturées à partir du set de capteurs EMG (703) ou la modification du régime de traitement de physiothérapie pour le CPP au serveur basé dans le nuage (715) pour communication avec un prestataire de santé.
